# EUROPÄISCHE PATENTANMELDUNG

(11) **EP 3 424 910 A1**
(43) Veröffentlichungstag der Anmeldung: **09.01.2019**
(21) Anmeldenummer: 17179716.0
(22) Anmeldetag: 05.07.2017
(51) Int. Cl.: C07D 251/34, C08G 18/79, C08G 18/02

(54) **KONTINUIERLICHE VERDÜNNUNG VON POLYISOCYANATEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung einer Polyisocyanatzusammensetzung, umfassend die Zugabe mindestens eines gegenüber Isocyanatgruppen inerten Lösungsmittels zu mindestens einem Polyisocyanat, dadurch gekennzeichnet, dass die Zugabe des Lösungsmittels in einer oder mehrerer Stufen erfolgt und mindestens eine dieser Stufen als kontinuierliche Verdünnung durchgeführt wird. Darüber hinaus betrifft die Erfindung die nach dem Verfahren erhältlichen Polyisocyanatzusammensetzungen, die Verwendung der Polyisocyanatzusammensetzungen, ein Zwei-Komponenten-System enthaltend die Polyisocyanatzusammensetzung und mit dem Zwei-Komponenten-System hergestellte Verbundsysteme.

## Beschreibung

Die vorliegende Erfindung betrifft ein Verfahren zur Herstellung einer Polyisocyanatzusammensetzung, insbesondere einer Polyisocyanatzusammensetzung aus Toluylendiisocyanat, und die daraus erhältlichen Polyisocyanatzusammensetzungen. Außerdem betrifft die Erfindung die Verwendung der Polyisocyanatzusammensetzung als Vernetzungsmittel in Lacken und Klebstoffen und die Verwendung einer kontinuierlichen Verdünnung. Weiterhin betrifft die vorliegende Erfindung ein, die Polyisocyanatzusammensetzung enthaltendes Zwei-Komponenten-System und die daraus erhältlichen Verbundsysteme.

Urethangruppen aufweisende Polyisocyanate aus Polyhydroxyverbindungen und Toluylendiisocyanat sind seit langem bekannt und werden z.B. in der DE 870 400, DE 953 012 und DE 1 090 196 beschrieben.

Isocyanurate des Toluylendiisocyanats werden durch Cyclotrimerisierung unter Verwendung verschiedener Katalysatoren hergestellt. Solche Umsetzungsprodukte sind ebenfalls seit langem bekannt und werden zum Beispiel in der DE 951168 B, DE 1013869 A, US 6,936,678 B2, DE 19523657 A1, US 4255569 A, EP 2 174 967 B1 und der CN 105001701 beschrieben.

Seit langem besteht der Wunsch, die bekannten Isocyanurate des Toluylendiisocyanats einerseits niedrig-viskos, andererseits hoch-funktionell darzustellen. Toluylendiisocyanat wird im Folgenden auch als TDI bezeichnet.

Eine niedrige Viskosität ist zum Beispiel erwünscht, um das Applikationsverhalten von Lacken und Klebstoffen zu verbessern. Daneben kann bei Verwendung von niedrig-viskosen Polyisocyanaten als Vernetzer von Lacken und Klebstoffen der Lösemittelgehalt der Formulierung gesenkt werden. Das bedeutet, dass die Emissionen flüchtiger organischer Verbindungen solcher Formulierungen reduziert werden können, ohne die Anwendbarkeit negativ zu beeinflussen.

Außerdem ist es beim Einsatz solcher Polyisocyanate als Vernetzer in Lacken und Klebstoffen erwünscht, dass die Polyisocyanate einen hohen Gehalt an Isocyanatgruppen aufweisen. Dadurch wird die Nachhaltigkeit im Sinne eines niedrigen Gehalts an organischen Lösungsmitteln und schneller Vernetzung, also hoher Prozesseffizienz, weiter gesteigert.

Daneben ist es erwünscht, dass die Isocyanurate des TDI einen niedrigen Gehalt an freiem Diisocyanat enthalten. Wegen der toxikologischen Bedenklichkeit des monomeren TDI ist dies eine wichtige Bedingung für die universelle Einsetzbarkeit in industriell applizierten Lacken und Klebstoffen.

Bei der Umsetzung von TDI zum Polyisocyanurat werden, wie aus DE 951168B, DE 1013869A bekannt, sehr hoch-viskose Harze erhalten, was die Verarbeitung erschwert oder es erforderlich macht, größere Mengen organischer Lösungsmittel zu verwenden. Daneben weisen Polyisocyanate des TDI eine hohe Tendenz zur Kristallisation auf und sind nur schwer in organischen Lösungsmitteln löslich.

Eine weitere wichtige Rahmenbedingung stellt die Löslichkeit der oligomeren Isocyanurate in gängigen organischen Lösungsmitteln dar. Eine unvollständige Löslichkeit führt zu trüben Lösungen, was die Verwendbarkeit in Lack- oder Klebstoff-Formulierungen stark einschränkt.

Aufgabe der vorliegenden Erfindung war es daher, ein Verfahren zur Herstellung einer Polyisocyanatzusammensetzung bereitzustellen, mit dem sich dem sich Polyisocyanatzusammensetzungen erhalten lassen, die eine niedrige Viskosität bei gleichzeitig möglichst hohem Gehalt an Isocyanatgruppen aufweisen.

Diese Aufgabe wurde gelöst durch Verfahren zur Herstellung einer Polyisocyanatzusammensetzung, umfassend die Zugabe mindestens eines gegenüber Isocyanatgruppen inerten Lösungsmittels zu mindestens einem Polyisocyanat, dadurch gekennzeichnet, dass die Zugabe des Lösungsmittels in einer oder mehrerer Stufen erfolgt und mindestens eine dieser Stufen als kontinuierliche Verdünnung durchgeführt wird.

Mit dem erfindungsgemäßen Verfahren lassen sich Polyisocyanatzusammensetzungen herstellen, die gegenüber dem Stand der Technik trübungsstabiler sind und daher auch für Verwendungen geeignet sind, in denen klare Lösungen benötigt werden.

Insbesondere lag der vorliegenden Erfindung die spezielle Aufgabe zugrunde ein Verfahren zur Herstellung einer Polyisocyanatzusammensetzung aus Toluylendiisocyanat bereitzustellen, mit dem sich dem Polyisocyanatzusammensetzungen aus Toluylendiisocyanat erhalten lassen, die eine niedrige Viskosität bei gleichzeitig möglichst hohem Gehalt an Isocyanatgruppen aufweisen und zudem als trübungsstabile Lösungen vorliegen.

Diese spezielle Aufgabe wurde gelöst durch ein Verfahren zur Herstellung einer Polyisocyanatzusammensetzung aus Toluylendiisocyanat, umfassend die Zugabe mindestens eines gegenüber Isocyanatgruppen inerten Lösungsmittels zu mindestens einem Polyisocyanat aus Toluylendiisocyanat, dadurch gekennzeichnet, dass die Zugabe des Lösungsmittels in einer oder mehrerer Stufen erfolgt und mindestens eine dieser Stufen als kontinuierliche Verdünnung durchgeführt wird.

Somit ist in einer ersten bevorzugten Ausführungsform das Polyisocyanat aus Toluyendiisocyanat. Der Begriff "aus Toluylendiisocyanat" ist gleichbedeutend mit "auf Basis von Toluylendiisocyanat".

Vorliegend steht Toluylendiisocyanat als Oberbegriff für die Isomere 2,4-Toluylendiisocyanat, 2,6-Toluylendiisocyanat und beliebige Gemische aus 2,4- und 2,6-Toluylendiisocyanat.

Erfindungsgemäß bedeuten die Bezugnahmen auf "umfassend", "enthaltend" usw. bevorzugt "im Wesentlichen bestehend aus" und ganz besonders bevorzugt "bestehend aus".

Vorliegend ist unter "kontinuierlicher Verdünnung" zu verstehen, das mindestens zwei Volumenströme, bevorzugt genau zwei Volumenströme, derart miteinander vermischt werden, dass die Verdünnung im Wesentlichen ohne Konzentrationsverlauf erfolgt. Unter "im Wesentlichen ohne Konzentrationsverlauf" wird vorliegend verstanden, dass der Festkörpergehalt im abfließenden Produktstrom zwischen ≤ 10% über und ≤ 10% unter, bevorzugt zwischen ≤ 5% über und ≤ 5% unter und besonders bevorzugt zwischen ≤ 2% über und ≤ 2% unter dem zu erreichenden Festkörpergehalt des verdünnten Produktstroms variieren kann.

Geeignete Vorrichtungen zur kontinuierlichen Verdünnung sind beispielsweise T-Stücke, Zweileitungssysteme mit Statikmischer sowie Kessel bzw. Zwischenlösekessel. Als Kessel bzw. Zwischenlösekessel wird vorliegend ein Behälter verstanden, in den mindestens zwei Volumenströme kontinuierlich in ein gerührtes Volumen gegeben werden, aus dem der verdünnte Produktstrom entsprechend abfließt. Dieser Produktstrom stellt die erfindungsgemäße Polyisocyanat-zusammensetzung dar, wobei beim Vorliegen mehrerer Verdünnungsstufen der, nach der letzten Verdünnungsstufe erhaltene Produktstrom die erfindungsgemäße Polyisocyanatzusammensetzung darstellt.

Bevorzugt erfolgt die kontinuierliche Verdünnung dadurch, dass mindestens zwei Flüssigkeitsströme, besonders bevorzugt genau zwei Flüssigkeitsströme, kontinuierlich in ein gerührtes Volumen gegeben werden, aus dem der verdünnte Produktstrom bevorzugt kontinuierlich abfließt. Ein solches gerührtes Volumen kann beispielsweise ein vorstehend genannter Kessel sein. In der Regel handelt es sich bei diesen Flüssigkeitsströmen zum einen um das zu lösende Polyisocyanat aus TDI (Polyisocyanatstrom) und zum anderen um das mindestens eine gegenüber Isocyanatgruppen inerte Lösungsmittel (Lösungsmittelstrom).

Im Gegensatz zur bekannten batch-Verdünnung können Nachteile bei der Durchmischung beim erfindungsgemäßen Verfahren weitestgehend vermieden werden, da stets direkt die gewünschte Konzentration vorliegt. Außerdem kann so die Verweilzeit so kurz wie möglich gehalten werden, was sich vorteilhaft auf die Stabilität der Produkte auswirkt.

Als Lösungsmittel können in der Polyurethanchemie gebräuchliche Verdünnungsmittel und Lösungsmittel wie beispielsweise Toluol, Xylol, Cyclohexan, Butylacetat, Ethylacetat, Ethylglykolacetat, Pentylacetat, Hexylacetat, Methoxypropylacetat, Tetrahydrofuran, Dioxan, Aceton, N-Methylpyrrolidon, Methylethylketon, Testbenzin, höher substituierte Aromaten, wie beispielsweise unter der Bezeichnung Solvent Naphtha®, Solvesso®, Shellsol®, Isopar®, Nappar® und Diasol® im Handel, Schwerbenzol, Tetralin, Dekalin und Alkane mit mehr als 6 Kohlenstoffatomen, übliche Weichmacher, wie Phthalate, Sulfonsäureester und Phosphorsäureester sowie Gemische derartiger Verdünnungs- und Lösungsmittel eingesetzt werden.

Ferner geeignet als Lösungsmittel sind auch Polyisocyanate auf Basis aliphatischer Diisocyanate wie sie z.B. in der DE-A 4 428 107 beschrieben sind. Hiermit sind verdünnte monomerenarme TDI-Trimerisate zugänglich, die keine oder weniger leicht verdampfbaren Lösungsmittel und Verdünnungsmittel enthalten.

In einer weiteren bevorzugten Ausführungsform des erfindungsgemäßen Verfahrens erfolgt die Zugabe des Lösungsmittels mindestens zweistufig, wobei die erste Stufe als kontinuierliche Verdünnung durchgeführt wird. Die mindestens erfolgende zweite Stufe kann kontinuierlich oder diskontinuierlich durchgeführt werden. Hieraus ergibt sich der Vorteil, dass die Trübungsstabilität der erfindungsgemäßen Polyisocyanatzusammensetzung noch weiter erhöht wird.

Es ist auch möglich, dass eine dritte, vierte, fünfte oder vielfache Stufe erfolgt, wobei hier zwischen erforderlichem verfahrenstechnischem Aufwand und der möglichem Zugewinn an weiterer Trübungsstabilität abgewogen werden muss. Hierbei hat sich herausgestellt, dass in den meisten Fällen eine zweistufige Zugabe die optimale Balance aus verfahrenstechnischem Aufwand und möglichem Zugewinn an weiterer Trübungsstabilität bringt.

Bei einer mehrstufigen bzw. mehrere Stufen umfassenden Zugabe mindestens eines gegenüber Isocyanatgruppen inerten Lösungsmittels werden in den sequenziell, in verschiedenen Vorrichtungen, wie beispielsweise Zwischenlösekesseln, ablaufenden Stufen verschiedene Verdünnungseinstellungen (Festkörpergehalte) erreicht. Diese Verdünnungseinstellungen werden im Folgenden auch als Festkörpergehalte bezeichnet. Vorliegend wird unter Festkörpergehalt der Gewichtsanteil des Polyisocyanats in der Polyisocyanatzusammensetzung verstanden.

In einer weiteren bevorzugten Ausführungsform des erfindungsgemäßen Verfahrens wird in der ersten Stufe ein Festkörpergehalt von ≥ 30 bis ≤ 90 Gew.-%, bevorzugt ≥ 50 bis ≤ 85 Gew.-%, besonders bevorzugt ≥ 55 bis ≤ 75 Gew.-% und ganz besonders bevorzugt ≥ 60 bis ≤ 70 Gew.-%, eingestellt.

Bei dieser mindestens zweistufig erfolgenden Zugabe ist es weiter bevorzugt, wenn in der zweiten Stufe ein Festkörpergehalt von ≥ 10 bis ≤ 80 Gew.-%, bevorzugt ≥ 15 bis ≤ 65 Gew.-%, besonders bevorzugt ≥ 20 bis ≤ 50 Gew.-% und ganz besonders bevorzugt ≥ 25 bis ≤ 35 Gew.-%, eingestellt wird, wobei der in der ersten Stufe eingestellte Festkörpergehalt in der zweiten Stufe zumindest um 15 Gew.-%, bevorzugt zumindest um 25 Gew.-% erniedrigt wird. Hieraus ergibt sich der Vorteil, dass sich über die mindestens zweistufige Zugabe Festkörpergehalte einstellen lassen, die für eine spätere Applikation der nach dem erfindungsgemäßen Verfahren erhältlichen Polyisocyanatzusammensetzung breitestmöglich geeignet sind und trotzdem trübungsstabil sind.

In einer weiteren bevorzugten Ausführungsform des erfindungsgemäßen Verfahrens wird das Polyisocyanat aus Toluylendiisocyanat durch
(i) Umsetzung von Toluylendiisocyanat unter Bildung eines Polyisocyanates und
(ii) Entfernung des nicht umgesetzten Toluylendiisocyanates hergestellt.

Die Entfernung des nicht umgesetzten Toluylendiisocyanates in Schritt (ii) kann nach beliebigen Methoden erfolgen. Es ist jedoch bevorzugt, wenn die Entfernung des nicht umgesetzten Toluylendiisocyanates in Schritt (ii) durch mindestens ein thermisches Trennverfahren, welches eine oder mehrere Stufen aufweisen kann, bevorzugt durch mindestens ein zweistufiges thermisches Trennverfahren und besonders bevorzugt durch mindestens einen Fallrohrverdampfer und/oder mindestens einen Dünnschichtverdampfer erfolgt. Dies führt zu dem Vorteil, dass eine ausreichende Abtrennung des nicht umgesetzten Toluylendiisocyanates auch bei größeren Durchsätzen erreicht werden kann.

Geeignete thermische Trennverfahren sind beispielsweise Destillationen im Vakuum mittels Dünnschichtverdampfer und/oder Fallrohrverdampfer. Für die Entfernung von TDI sind im Allgemeinen Drücke im Bereich von 0,1-20 mbar und Temperaturen von 120-250 °C geeignet.

Bevorzugt wird das thermische Trennverfahren bei einer Heizmitteltemperatur von ≥ 140 bis ≤ 235 °C und bevorzugt von ≥ 160 bis ≤ 215 °C durchgeführt. Hieraus ergibt sich der Vorteil, dass die Entfernung des nicht umgesetzten Toluylendiisocyanats schonend und dennoch effizient erfolgt. Je nach verfahrenstechnischem Aufwand lassen sich Gehalte an monomerem Toluylendiisocyanat von ≤ 0,5 Gew.-%, bevorzugt ≤ 0,3 Gew.-% und besonders bevorzugt ≤ 0,1 Gew.-%, bezogen auf das Gesamtgewicht des Polyisocyanats aus Toluylendiisocyanat realisieren, je niedriger diese Gehalte sind, desto breiter ist die erfindungsgemäße Polyisocyanatzusammensetzung einsetzbar, da die Arbeitsplatzhygiene, insbesondere in handwerklichen Applikationen, noch weiter verbessert wird. Bestimmen lassen sich die Gehalte an nicht umgesetzten Toluylendiisocyanat gaschromatographisch nach DIN EN ISO 10283:2007-11 mit internem Standard.

In einer weiteren bevorzugten Ausführungsform wird in Schritt (i) des Verfahrens ein Gemisch aus 2,4- und 2,6-Toluylendiisocyanat eingesetzt, welches zu ≥ 50 bis ≤ 99 Gew.-%, bevorzugt zu ≥ 70 bis ≤ 95 Gew.-% und besonders bevorzugt zu ≥ 75 bis ≤ 90 Gew.-%, bezogen auf das Gesamtgewicht des eingesetzten Toluylendiisocyanates, aus 2,4-Toluylendiisocyanat besteht. Hieraus ergibt sich der weitere Vorteil, dass eine ausgewogene Balance zwischen Selektivität der unterschiedlich reaktiven Isocyanatgruppen im 2,4-TDI und Erhöhung der Kristallisationsstabilität durch zumindest einen kleinen Anteil an 2,6-TDI erreicht wird.

Sowohl 2,4- und 2,6-Toluylendiisocyanat als auch deren Gemische sind allgemein kommerziell verfügbar. Sie können nach bekannten Verfahren, wie beispielsweise durch Phosgenierung des entsprechenden Toluylendiamins (TDA) in der Flüssigphase oder der Gasphase hergestellt werden. Besonders bevorzugt sind Toluylendiisocyanate, die durch Gasphasenphosgenierung von TDA hergestellt werden, da ein solches Verfahren besonders effizient ist.

In einer weiteren Ausführungsform ist das in Schritt (i) des Verfahrens gebildete Polyisocyanat ein Urethanguppen aufweisendes Polyisocyanat aus Toluylendiisocyanat. Dieses wird vorzugsweise so hergestellt, dass Polyhydroxyverbindungen mit der 5- bis 10-fachen molaren Menge TDI umgesetzt werden. Hierbei sind geeignete niedermolekulare Polyhydroxyverbindungen zwei- bis vierwertige Alkohole mit einem Molekulargewicht von 62 bis 146 und/oder aus ihnen durch Addition von Ethylen- und/oder Propylenoxid hergestellte Polyetherpolyole in reiner Form oder als beliebige Gemische.

Als zwei- bis vierwertige Alkohole kommen zum Beispiel in Betracht Ethylenglykol, 1,2-Propylenglykol, 1,3-Propylenglykol, 1,2-Butandiol, 1,3-Butandiol, 1,4-Butandiol, 1,5-Pentandiol, Neopentylglykol, 1,6-Hexandiol, 2-Ethylhexandiol, Glycerin, Trimethylolpropan und Pentaerythrit.

Geeignete Polyetherpolyole weisen ein aus Hydroxylgruppengehalt und Hydroxylfunktionalität berechenbares Molekulargewicht von 106 bis 600, vorzugsweise 106 bis 470 auf. Bevorzugt werden Polyetherdiole und Polyethertriole eingesetzt. Diese Polyetherpolyole können in an sich bekannter Weise durch Alkoxylierung geeigneter zwei- bis vierfunktioneller Startermoleküle oder geeigneter Gemische von Startermolekülen erhalten werden, wobei bei der Alkoxylierung insbesondere Propylenoxid und/oder Ethylenoxid, gegebenenfalls im Gemisch nacheinander in beliebiger Reihenfolge zum Einsatz gelangen. Bevorzugt werden als Startermoleküle die oben genannten zwei- bis vierwertigen Alkohole eingesetzt. Ganz besonders bevorzugt werden Gemische aus Trimethylolpropan und Diethylenglykol eingesetzt.

In einer weiteren bevorzugten Ausführungsform ist das Polyisocyanat des erfindungsgemäßen Verfahrens ein Isocyanatgruppen aufweisendes Polyisocyanurat aus Toluylendiisocyanat, wobei die Umsetzung von Toluylendiisocyanat in Schritt (i) unter Bildung von Isocyanuratgruppen in Anwesenheit mindestens eines Katalysators erfolgt und bei einem Gehalt an Isocyanatgruppen von ≥ 30 bis ≤ 46 Gew.-%, bevorzugt von ≥ 34 bis ≤ 44 Gew.-% und besonders bevorzugt von ≥ 38 bis ≤ 42 Gew.-% durch Zugabe mindestens eines Katalysatorgiftes abgestoppt wird.

Bei dieser Ausführungsform werden die Schritte (i) und (ii) vorzugsweise in Anwesenheit von ≥ 0 bis < 1 Gew.-% bei Destillationsbedingungen inerten, flüssigen, mindestens 50 °C über dem Toluylendiisocyanat siedenden Destillationshilfsmitteln und/oder ≥ 0 bis < 1 Gew.-%, bezogen auf das Gesamtgewicht der in Schritt (i) eingesetzten Verbindungen, an Verbindungen, die eine oder mehrere Hydroxylgruppen aufweisen, durchgeführt.

Besonders bevorzugt ist hierbei, dass solche Destillationshilfsmittel in Mengen von ≥ 0 bis ≤ 0,5 Gew.-%, bevorzugt ≥ 0 bis ≤ 0,25 Gew.-% und besonders bevorzugt ≥ 0 bis ≤ 0,1 Gew.-%, bezogen auf das Gesamtgewicht der in Schritt (i) eingesetzten Verbindungen, vorhanden sind und/oder, dass die Verbindungen, die eine oder mehrere Hydroxylgruppen aufweisen in Mengen von ≥ 0 bis ≤ 0,8 Gew.-%, bevorzugt ≥ 0 bis ≤ 0,5 Gew.-%, besonders bevorzugt ≥ 0 bis ≤ 0,1 Gew.-%, bezogen auf das Gesamtgewicht der in Schritt (i) eingesetzten Verbindungen, vorhanden sind. In diesen Mengen gegebenenfalls vorhandene Destillationshilfsmittel und/oder in diesen Mengen gegebenenfalls vorhandene Verbindungen, die eine oder mehrere Hydroxylgruppen aufweisen, haben keinen negativen Einfluss auf das erfindungsgemäße Verfahren. Jedoch ist es ganz besonders bevorzugt, wenn in den Schritten (i) und (ii) des erfindungsgemäßen Verfahrens keine Destillationshilfsmittel und/oder keine Verbindungen, die eine oder mehrere Hydroxylgruppen aufweisen, vorhanden sind, mit Ausnahme der gegebenenfalls vorhandenen, nachfolgend als Katalysatorbestandteil genannten aromatischen Hydroxylgruppen.

Für den Fall, dass gegenüber Isocyanatgruppen inerte Lösungsmittel bei dem erfindungsgemäßen Verfahren in den Schritten (i) bis (ii) zugegen sind, ist bevorzugt, dass solche Lösungsmittel in den Schritten (i) bis (ii) zu ≥ 0 bis ≤ 3 Gew.-%, bevorzugt ≥ 0 bis ≤ 1 Gew.-% und besonders bevorzugt ≥ 0 bis ≤ 0,05 Gew.-%, bezogen auf das Gesamtgewicht der in Schritt (i) eingesetzten Verbindungen, anwesend sein können.

Als Katalysatoren für die Bildung von Isocyanuratgruppen, folgend auch als Trimerisierungskatalysatoren bezeichnet, kommen grundsätzlich alle bekannten Katalysatoren des Standes der Technik wie beispielsweise Phosphine, Alkalisalze, Alkalialkoholate, tertiäre Amine, Fluoride, Hydrogendifluoride oder Hydrogenpolyfluoride in Betracht. Vorzugsweise werden Katalysatoren eingesetzt, die an Aromaten gebundene N,N-Dialkylaminomethylgruppen und phenolische OH-Gruppen aufweisen (Alkyl: unabhängig Alkylkette oder Alkylenkette mit bis 18 Kohlenstoffatomen, die gegebenenfalls durch Sauerstoff oder Schwefel getrennt sind). Diese Gruppen können auf mehrere Moleküle verteilt oder an einem oder mehreren benzolischen Aromaten positioniert sein. Besonders bevorzugt werden Katalysatoren eingesetzt, die sowohl Hydroxyl- als auch Dialkylaminomethylgruppen in einem Molekül enthalten. Ganz besonders bevorzugt werden Katalysatoren eingesetzt, deren Dialkylaminomethylgruppen (Alkyl = C₁ bis C₃-Kette) in ortho-Stellung zu aromatischen Hydroxylgruppen positioniert sind. Als Beispiele seien folgende Mannichbasen genannt, wie sie z.B. auf Basis von Phenol, p-Isononylphenol oder Bisphenol A zum Beispiel durch Umsetzung von 188 Gewichtsteilen Phenol mit 720 Teilen einer 25%igen wässrigen Dimethylamin-Lösung und 425 Gewichtsteilen 40%iger Formaldehyd-Lösung durch zweistündiges Erhitzen auf 80°C, Abtrennen der wässrigen Phasen und Destillation der organischen Phase bei 90°C/10 Torr nach der DE-A 2 452 531 9 erhalten werden.

Die Umsetzung in Schritt (i) erfolgt im Allgemeinen bei Temperaturen zwischen 20 und 120°C, bevorzugt zwischen 40 und 100°C und besonders bevorzugt zwischen 60 und 90°C.

Die Katalysatoren werden als Reinsubstanz oder gelöst gegebenenfalls in mehreren kleinen Portionen in Schritt (i) eingesetzt, wobei die Menge über einen breiten Bereich variiert werden kann. Vorzugsweise beträgt die Menge an insgesamt eingesetztem Katalysator ≥ 0,001 bis ≤ 2,0 Gew.-%, bevorzugt ≥ 0,003 bis ≤ 0,5 Gew.-% und besonders bevorzugt ≥ 0,005 bis ≤ 0,05 Gew.-%, bezogen auf das Gesamtgewicht der in Schritt (i) und (ii) eingesetzten Verbindungen.

Das Abstoppen der Umsetzung in Schritt (i) erfolgt durch Zugabe mindestens eines Katalysatorengifts, wobei als Katalysatorengifte beispielsweise Schwefel (im Falle der Verwendung von Phosphinen als Katalysatoren) oder Alkylierungsmittel wie beispielsweise Toluolsulfonsäuremethylester (bei der bevorzugten Verwendung von Mannich-Basen als Katalysatoren) oder auch Acylierungsmittel wie beispielsweise Benzoylchlorid Verwendung finden können.

Die Menge des einzusetzenden Katalysatorgiftes wird entsprechend der eingesetzten Katalysatormenge ausgewählt, so dass der Katalysator deaktiviert wird. Vorzugsweise wird eine insgesamt unteräquimolare Menge des Katalysatorgifts bezogen auf die Äquivalente an Lewis-Basen der Katalysatoren eingesetzt, können auch bereits > 20 bis < 100%, bezogen auf die eingesetzten Äquivalente an Lewis-Base des Katalysators, für eine vollständige Deaktivierung des Katalysators ausreichend sein.

Unabhängig davon welches Polyisocyanat aus TDI in dem erfindungsgemäßen Verfahren eingesetzt wird, erfolgt in einer weiteren bevorzugten Ausführungsform nach Zugabe des mindestens einen gegenüber Isocyanatgruppen inerten Lösungsmittels zu dem mindestens einem Polyisocyanat, bevorzugt aus Toluylendiisocyanat, in einem weiteren Schritt die Zugabe mindestens einer vom Polyisocyanat der vorstehenden Ausführungsformen verschiedenen Polyisocyanatzusammensetzung aus Toluylendiisocyanat, bevorzugt mindestens einer Polyisocyanuratzusammensetzung aus Toluylendiisocyanat und/oder mindestens einer Polyurethanzusammensetzung aus Toluylendiisocyanat, sowie optional die Zugabe eines oder mehrerer Hilfs- und Zusatzstoffe. Diese Polyisocyanuratzusammensetzung aus Toluylendiisocyanat und diese Polyurethanzusammensetzung aus Toluylendiisocyanat lassen sich nach bekannten Verfahren herstellen, aber auch nach den vorstehend beschriebenen Verfahren zur Herstellung des Isocyanatgruppen aufweisenden Polyisocyanurats aus Toluylendiisocyanat und des Urethanguppen aufweisendes Polyisocyanat aus Toluylendiisocyanat.

Hieraus ergibt sich der weitere Vorteil, dass die physikalischen und chemischen Eigenschaften von Gemischen, enthaltend mindestens ein erfindungsgemäßes Polyisocyanat, gezielt einstellen lassen.

Geeignete Hilfs- und Zusatzstoffe sind beispielsweise die üblichen Benetzungsmittel, Verlaufsmittel, Hautverhinderungsmittel, Antischaummittel, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden.

Eine Polyisocyanatzusammensetzung, hergestellt oder herstellbar nach dem erfindungsgemäßen Verfahren ist ein weiterer Gegenstand der Erfindung, da sich überraschenderweise gezeigt hat, dass eine solche Verfahrensführung zu trübungsstabilen Zusammensetzungen führt, wohingegen eine Zugabe von Lösungsmittel, bei der nicht mindestens eine Stufe als kontinuierliche Verdünnung durchgeführt wird zu getrübten Zusammensetzungen führt.

Ein weiterer Gegenstand der Erfindung ist die Verwendung der kontinuierlichen Verdünnung bei der Lösung von Polyisocyanaten, bevorzugt von Polyisocyanaten aus Toluylendiisocyanat, zur Verhinderung von Trübungen in der Polyisocyanatzusammensetzung.

Die erfindungsgemäße Polyisocyanatzusammensetzung ist sehr gut zur Verwendung als Vernetzungsmittel in einem Klebstoff oder in einem Beschichtungsmittel geeignet. Daher ist dies ein weiterer Gegenstand der Erfindung.

Die durch das erfindungsgemäße Verfahren herstellbaren Polyisocyanatzusammensetzungen werden vorzugsweise zur Herstellung von unter dem Einfluss von Luftfeuchtigkeit aushärtbaren Klebstoffen oder Beschichtungsmaterialien verwendet. Sie können ebenfalls zur Herstellung von Haftvermittlern, Druckfarben und Polyurethanformkörpern Verwendung finden. Besonders bevorzugt werden sie als Vernetzer in Zwei-Komponenten-Systemen mit an sich bekannten gegenüber Isocyanatgruppen reaktiven Verbindungen eingesetzt.

Ein weiterer Gegenstand der Erfindung ist daher ein Zwei-Komponenten-System, umfassend eine Isocyanatkomponente A), enthaltend mindestens eine erfindungsgemäße Polyisocyanatzusammensetzung, und eine gegenüber Isocyanatgruppen reaktive Komponente B), enthaltend mindestens eine gegenüber Isocyanatgruppen reaktive Verbindung, bevorzugt mindestens einen Hydroxylgruppen aufweisenden Polyester.

Geeignete gegenüber Isocyanatgruppen reaktive Verbindungen sind beispielsweise hydroxyfunktionelle Polyether, -ester, -amide, -carbonate, -acrylate, -butadiene bzw. Mischtypen der genannten hydroxyfunktionellen Polymeren. Auch niedermolekulare Di- und Polyole, Di- und Trimerfettalkohole sowie aminofunktionelle Verbindungen können in dem erfindungsgemäßen Zwei-Komponenten-System Verwendung finden. Außerdem sind auch Cyclohexanon-Formaldehyd-Kondensate, beispielsweise in Ricinusöl, geeignet. Hydroxylgruppen aufweisende Polyester sind jedoch besonders bevorzugt. Zusätzlich können in den Beschichtungen oder Verklebungen auch andere Hilfs- und Zusatzstoffe wie beispielsweise die üblichen Benetzungsmittel, Verlaufsmittel, Hautverhinderungsmittel, Antischaummittel, Haftvermittler, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden. Die Beschichtungsmittel können in Form von Klarlacken als auch in Form pigmentierter Lacke verwendet werden.

Die erhaltenen Beschichtungsmaterialien oder Klebstoffe können zur Beschichtung oder Verklebung beliebiger Substrate wie beispielsweise natürliche oder künstliche Faserstoffe, bevorzugt Holz, Kunststoffe, Leder, Papier, Textilien, Glas, Keramik, Putz, Mauerwerk, Metalle oder Beton und besonders bevorzugt Papier oder Leder verwendet werden. Sie lassen sich mit üblichen Applikationsmethoden wie Spritzen, Streichen, Fluten, Gießen, Tauchen, Walzen aufbringen.

Daher ist ein Verbundsystem, hergestellt oder herstellbar durch Aushärten des auf mindestens ein Substrat aufgetragenen erfindungsgemäßen Zwei-Komponenten-Systems ein weiterer Gegenstand der Erfindung.

Die Erfindung wird nachfolgend anhand von Beispielen und Vergleichsbeispielen näher erläutert, ohne sie jedoch auf diese einzuschränken.

### Beispiele

Alle Prozentangaben beziehen sich, soweit nichts Anderslautendes vermerkt, auf das Gewicht.

Die Bestimmung der NCO-Gehalte erfolgte titrimetrisch nach DIN EN ISO 11909:2007-05.

Die Rest-Monomeren Gehalte wurden nach DIN EN ISO 10283:2007-11 gaschromatographisch mit internem Standard gemessen.

Die Trübung wurde nach DIN EN ISO 7027-1:2016 gemessen.

### Beispiel 1 (nicht erfindungsgemäß):

1500 g einer Toluylendiisocyanat-Isomerenmischung aus ca. 80% 2,4-Toluylendiisocyanat und 20% 2,6- Toluylendiisocyanat werden bei 80°C in einem 2L-Kolben vorgelegt. Dann werden 0,52 g einer Mannichbase (Bisphenol/Formaldehyd/Dimethylamin 25%ig in Butylacetat / Xylol 19:56) innerhalb von 2 Stunden unter Rühren zudosiert, wobei die Temperatur bei 78-82°C gehalten wird. Wenn ein NCO-Gehalt 40,4% erreicht ist, wird 1 g Dibutylphosphat zum Abstoppen der Reaktion zugegeben. Aus der so erhaltenen Rohware wird das überschüssige Diisocyanat anschließend bei Temperaturen von 180°C und einem Druck von 0,05 mbar kontinuierlich destillativ entfernt. Das erhaltene heiße Harz (370 g) wird mittels einer Pumpe direkt in einen gerührten Kolben, ausgestattet mit einem Rückflusskühler, zu 863 g siedendem Ethylacetat gefördert, was einer einstufigen, diskontinuierlichen Verdünnung von 100% Festkörpergehalt auf 30% Festkörpergehalt entspricht. Man erhält eine Lösung mit folgenden Eigenschaften:
NCO: 7,1%
Monomerengehalt: 0,08%
Trübung: 21 NTU

### Beispiel 2 (erfindungsgemäß):

1500 g einer Toluylendiisocyanat-Isomerenmischung aus ca. 80% 2,4-Toluylendiisocyanat und 20% 2,6- Toluylendiisocyanat werden bei 80°C in einem 2L-Kolben vorgelegt. Dann werden 0,52 g einer Mannichbase (Bisphenol/Formaldehyd/Dimethylamin 25%ig in Butylacetat / Xylol 19:56) innerhalb von 2 Stunden unter Rühren zudosiert, wobei die Temperatur bei 78-82°C gehalten wird. Wenn ein NCO-Gehalt 40,4% erreicht ist, wird 1 g Dibutylphosphat zum Abstoppen der Reaktion zugegeben. Aus der so erhaltenen Rohware wird das überschüssige Diisocyanat anschließend bei Temperaturen von 180°C und einem Druck von 0,05 mbar kontinuierlich destillativ entfernt. Das aus der Destillation ablaufende heisse Harz wird mit 105 g/h mittels einer Pumpe direkt in einen gerührten 500ml-4-Hals-Kolben, ausgestattet mit einem Rückflusskühler, gefördert. Zeitgleich läßt man aus einem Tropftrichter 245 g/h Ethylacetat zulaufen und überführt das kontinuierlich verdünnte Produkt über ein getauchtes Steigrohr mittels einer Pumpe mit 350 g/h kontinuierlich in einen gekühlten Vorlagekolben. Der Inhalt des 4-Hals-Kolbens bleibt bei ca. 400 g konstant hat eine Temperatur von ca. 80°C. Man erhält eine Lösung mit einem Festkörpergehalt von 30% und folgenden Eigenschaften:
NCO: 7,0%
Monomerengehalt: 0,03%
Trübung: 12 NTU

### Beispiel 3 (erfindungsgemäß):

1500 g einer Toluylendiisocyanat-Isomerenmischung aus ca. 80% 2,4-Toluylendiisocyanat und 20% 2,6- Toluylendiisocyanat werden bei 80°C in einem 2L-Kolben vorgelegt. Dann werden 0,52 g einer Mannichbase (Bisphenol/Formaldehyd/Dimethylamin 25%ig in Butylacetat / Xylol 19:56) innerhalb von 2 Stunden unter Rühren zudosiert, wobei die Temperatur bei 78-82°C gehalten wird. Wenn ein NCO-Gehalt 40,4% erreicht ist, wird 1 g Dibutylphosphat zum Abstoppen der Reaktion zugegeben. Aus der so erhaltenen Rohware wird das überschüssige Diisocyanat anschließend bei Temperaturen von 180°C und einem Druck von 0,05 mbar kontinuierlich destillativ entfernt. Das aus der Destillation ablaufende heisse Harz wird mit 105 g/h mittels einer Pumpe direkt in einen gerührten 500ml-4-Hals-Kolben, ausgestattet mit einem Rückflusskühler, gefördert. Zeitgleich läßt man aus einem Tropftrichter 57 g/h Ethylacetat zulaufen und überführt das kontinuierlich verdünnte Produkt über ein getauchtes Steigrohr mittels einer Pumpe mit 162 g/h kontinuierlich in einen gekühlten Vorlagekolben. Der Inhalt des 4-Hals-Kolbens bleibt bei ca. 400 g konstant hat eine Temperatur von ca. 80°C.

Anschließend wird der Inhalt des Vorlagekolbens in einem diskontinuierlichen Schritt mit Ethylacetat auf 30% Festkörper verdünnt. Man erhält eine Lösung mit folgenden Eigenschaften:
NCO: 7,0%
Monomerengehalt: 0,05%
Trübung: 2 NTU

## Patentansprüche

1. Verfahren zur Herstellung einer Polyisocyanatzusammensetzung, umfassend die Zugabe mindestens eines gegenüber Isocyanatgruppen inerten Lösungsmittels zu mindestens einem Polyisocyanat, **dadurch gekennzeichnet, dass** die Zugabe des Lösungsmittels in einer oder mehrerer Stufen erfolgt und mindestens eine dieser Stufen als kontinuierliche Verdünnung durchgeführt wird.

2. Verfahren gemäß Anspruch 1, **dadurch gekennzeichnet, dass** das Polyisocyanat aus Toluyendiisocyanat ist.

3. Verfahren gemäß Anspruch 1 oder 2, **dadurch gekennzeichnet, dass** die Zugabe des Lösungsmittels mindestens zweistufig erfolgt, wobei die erste Stufe als kontinuierliche Verdünnung durchgeführt wird.

4. Verfahren gemäß einem der vorstehenden Ansprüche, **dadurch gekennzeichnet, dass** in der ersten Stufe ein Festkörpergehalt von ≥ 30 bis ≤ 90 Gew.-%, bevorzugt ≥ 50 bis ≤ 85 Gew.-%, besonders bevorzugt ≥ 55 bis ≤ 75 Gew.-% und ganz besonders bevorzugt ≥ 60 bis ≤ 70 Gew.-%, eingestellt wird.

5. Verfahren gemäß einem der Anspruch 3 oder 4, **dadurch gekennzeichnet, dass** in der zweiten Stufe ein Festkörpergehalt von ≥ 10 bis ≤ 80 Gew.-%, bevorzugt ≥ 15 bis ≤ 65 Gew.-%, besonders bevorzugt ≥ 20 bis ≤ 50 Gew.-% und ganz besonders bevorzugt ≥ 25 bis ≤ 35 Gew.-%, eingestellt wird, wobei der in der ersten Stufe eingestellte Festkörpergehalt in der zweiten Stufe zumindest um 15 Gew.-%, bevorzugt zumindest um 25 Gew.-% erniedrigt wird.

6. Verfahren gemäß einem der vorstehenden Ansprüche, **dadurch gekennzeichnet, dass** das Polyisocyanat aus Toluylendiisocyanat durch
(i) Umsetzung von Toluylendiisocyanat unter Bildung eines Polyisocyanates und
(ii) Entfernung des nicht umgesetzten Toluylendiisocyanates hergestellt wird.

7. Verfahren gemäß Anspruch 6, **dadurch gekennzeichnet, dass** die Entfernung des nicht umgesetzten Toluylendiisocyanates in Schritt (ii) durch mindestens ein thermisches Trennverfahren, bevorzugt durch mindestens ein zweistufiges thermisches Trennverfahren und besonders bevorzugt durch mindestens einen Fallrohrverdampfer und/oder mindestens einen Dünnschichtverdampfer erfolgt.

8. Verfahren gemäß Anspruch 7, **dadurch gekennzeichnet, dass** das mindestens eine thermische Trennverfahren bei einer Heizmitteltemperatur von ≥ 140 bis ≤ 235 °C und bevorzugt von ≥ 160 bis ≤ 215 °C durchgeführt wird.

9. Verfahren gemäß einem der Ansprüche 6 bis 8, **dadurch gekennzeichnet, dass** das Polyisocyanat aus Toluylendiisocyanat ein Isocyanatgruppen aufweisendes Polyisocyanurat aus Toluylendiisocyanat ist, wobei die Umsetzung von Toluylendiisocyanat in Schritt (i) unter Bildung von Isocyanuratgruppen in Anwesenheit mindestens eines Katalysators erfolgt und bei einem Gehalt an Isocyanatgruppen von ≥ 30 bis ≤ 46 Gew.-%, bevorzugt von ≥ 34 bis ≤ 44 Gew.-% und besonders bevorzugt von ≥ 38 bis ≤ 42 Gew.-% durch Zugabe mindestens eines Katalysatorgiftes abgestoppt wird.

10. Verfahren gemäß einem der Ansprüche 6 bis 9, **dadurch gekennzeichnet, dass** die Schritte (i) und (ii) in Anwesenheit von ≥ 0 bis < 1 Gew.-% bei Destillationsbedingungen inerten, flüssigen, mindestens 50 °C über Toluylendiisocyanat siedenden Destillationshilfsmitteln und/oder ≥ 0 bis < 1 Gew.-%, bezogen auf das Gesamtgewicht der in Schritt (i) und (ii) eingesetzten Verbindungen, an Verbindungen, die eine oder mehrere Hydroxylgruppen aufweisen, durchgeführt werden.

11. Verfahren gemäß einem der vorstehenden Ansprüche, **dadurch gekennzeichnet, dass** nach Zugabe des mindestens einen gegenüber Isocyanatgruppen inerten Lösungsmittels zu dem mindestens einen Polyisocyanat in einem weiteren Schritt die Zugabe mindestens einer vom Polyisocyanat der vorstehenden Ansprüche verschiedenen Polyisocyanatzusammensetzung aus Toluylendiisocyanat, bevorzugt mindestens einer Polyisocyanuratzusammensetzung aus Toluylendiisocyanat und/oder mindestens einer Polyurethanzusammensetzung aus Toluylendiisocyanat, sowie optionale Zugabe eines oder mehrerer Hilfs- und Zusatzstoffe erfolgt.

12. Polyisocyanatzusammensetzung, hergestellt oder herstellbar gemäß einem Verfahren der Ansprüche 1 bis 11.

13. Verwendung der kontinuierlichen Verdünnung bei der Lösung von Polyisocyanaten, bevorzugt von Polyisocyanaten aus Toluylendiisocyanat, zur Verhinderung von Trübungen in der Polyisocyanatzusammensetzung.

14. Verwendung einer Polyisocyanatzusammensetzung gemäß Anspruch 12 als Vernetzungsmittel in einem Klebstoff oder einem Beschichtungsmittel.

15. Zwei-Komponenten-System umfassend eine Isocyanatkomponente A), enthaltend mindestens eine Polyisocyanatzusammensetzung gemäß Anspruch 12, und eine gegenüber Isocyanatgruppen reaktive Komponente B), enthaltend mindestens eine gegenüber Isocyanatgruppen reaktive Verbindung, bevorzugt mindestens einen Hydroxylgruppen aufweisenden Polyester.

16. Verbundsystem, hergestellt oder herstellbar durch Aushärten des auf mindestens ein Substrat aufgetragenen Zwei-Komponenten-Systems gemäß Anspruch 15.
